# EUROPEAN PATENT SPECIFICATION

(11) **EP 0 495 351 B1**
(45) Date of publication and mention of the grant of the patent: **20.12.1995**
(21) Application number: 92100009.7
(22) Date of filing: 02.01.1992
(51) Int. Cl.: A61B 1/00

(54) **Position measuring device for endoscope**
Positionsmesseinrichtung für Endoskop
Dispositif de mesure de position pour endoscope

(30) Priority: 14.01.1991 JP 4431/91
(43) Date of publication of application: 22.07.1992
(73) Proprietor: Hatano, Tadashi, Naha-shi, Okinawa-ken (JP)
(72) Inventor: Hatano, Tadashi, Naha-shi, Okinawa-ken (JP)
(74) Representative: Kraus, Walter, Dr.

(56) References cited:
- EP-A- 0 376 873
- WO-A-89/07909
- DE-U- 8 910 316
- US-A- 4 763 652

## Description

This invention relates to a measuring device, comprising:
(a) a plurality of arms successively connected to a fixed arm so that a front end of the connected plurality of arms is movable three-dimensionally;
(b) angle sensors provided in individual arm joints; and
(c) a computer to which the length of the individual arms previously measured and the angles sensed by the angle sensors are inputted.

Furthermore, this invention relates to a measuring method, comprising:
(a) measuring the length of a plurality of arms successively connected to a fixed arm so that a front end of the connected plurality of arms is movable three-dimensionally;
(b) measuring the angles between the individual arms by angle sensors connected to individual joints; and
(c) inputting to a computer the length of the individual arms previously measured and the angles sensed by the angle sensors.

A measuring device and method of the aforementioned types are described in US-A-4 763 652 using an ultrasonic sensor attached to the free end of the plurality of the connected arms for measuring the position of a kidney stone and the like.

Heretofore, various types of endoscopes have been used for observing spaces in the human body, that is, the inner surface of hollow organs and the inside of the thoracic cavity and abdominal cavity. The endoscope plays an important role in diagnosis since it enables visual observation of inside of the body from outside.

However, endoscopes, although having advantages of enabling direct visual identification of the presence and shape of an object within the body such as tumors, have had inconveniences in that they cannot numerically measure the size and position of the observed object. For example, the size of a tumor observed by the endoscope is largely affected by the distance between the tumor and the endoscope, and detailed and objective data cannot be obtained.

It is a primary object of the present invention to provide a device for observing an object using an endoscope and numerically measuring the size and position of the object.

### Disclosure of the Invention

In accordance with the present invention, there is provided a measuring device of the type mentioned in the beginning, which is characterized in that
(1) a direct-access endoscope is connected to the front end of the connected arms;
(2) the computer comprises means for storing the length of the direct-access endoscope previously measured; and
(3) the computer further comprises means for numerically determining the three-dimensional positions of the front end of the endoscope from the lengths of the individual arms, the angles between the individual arms and the length of the direct-access endoscope and thereby to determine the numerical size and/or position and/or area and/or distance from a specific portion of an object observed by the endoscope.

Furthermore, in accordance with the present invention, there is provided a measuring method of the type mentioned in the beginning, which is characterized by
(1) measuring the length of a direct-access endoscope connected to the front end of the arms;
(2) inputting the length of the direct-access endoscope previously measured to the computer;
(3) numerically determining the three-dimensional positions of the front end of the endoscope and/or the numerical size and/or position and/or area and/or distance by the computer from a specific portion of an object observed by the endoscope by:
   (i) aligning the front end of the endoscope with one portion of the object to be measured and calculating the numerical three-dimensional position of that one portion;
   (ii) aligning the front end of the endoscope with another portion of the object to be measured or a specific portion and calculating the numerical three-dimensional position of that portion;
   (iii)possibly repeating the steps (i) and (ii) for measuring the three-dimensional position of further portions of the object to be measured and/or the three-dimensional position of a specific portion; and
   (iv) calculating the numerical size and/or area and/or position of said object and/or the numerical distance of said object from said specific portion.

To make the front end of the arms after connection movable three-dimensionally, the plurality of arms are successively connected to the fixed arm using at least one joint which is rotatable in the horizontal direction relative to the vertical axis of arm and at least one joint which is rotatable in the vertical axial direction.

Alternatively, to make the front end of the arms movable three-dimensionally, for example, two rails are individually provided with sliders, the sliders are connected by two rails, a base plate is slidable mounted to the rails connecting the sliders, and a vertically expandable, rotatable arm may be mounted under the base plate.

### Brief Description of the Drawing

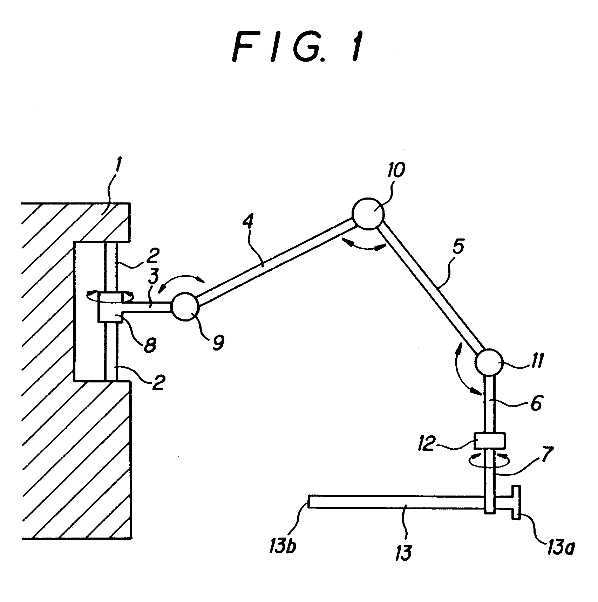

Fig.1 is a schematic view of the position measuring device for an endoscope according to the present invention.

### Best Mode for Practicing the Invention

The present invention will now be described in detail with reference to Fig.1 showing an embodiment. In Fig.1, numeral 1 indicates a fixture, numeral 2 indicates a fixed arm, numerals 3, 4, 5, 6, and 7 indicate arms, numerals 8, 9, 10, 11, and 12 indicate angle sensors provided at individual arm joints, numeral 13 indicates a direct-access endoscope, numeral 13a indicates an ocular, and numeral 13b indicates a front end.

The fixed arm 2 is mounted to the fixture 1. One end of the arm 3 is connected to the fixed arm 2 so that the arm 3 is rotatable in the horizontal direction relative to the vertical axis of the fixed arm 2. The other end of the arm 3 is connected to one end of the arm 4 so that the arm 4 is rotatable in the vertical axis direction of the arm 3. The other end of the arm 4 is connected to one end of the arm 5 so that the arm 5 is rotatable in the vertical axis direction of the arm 4. The other end of the arm 5 is connected to one end of the arm 6 so that the arm 6 is rotatable in the vertical direction of the arm 5. Furthermore, the other end of the arm 6 is connected to one end of the arm 7 so that the arm 7 is rotatable in the horizontal direction relative to the vertical axis of the arm 6. The rotatable connection is achieved using conventional means known in the art. The direct-access endoscope 13 is vertically attached to the other end of the arm 7, and the angle sensors 8, 9, 10, 11, and 12, which are of a conventional type known in the art, are attached to the individual joints.

Since the individual joints between the fixed arm and the arm 3 and between the arm 6 and the arm 7 are rotatable in the horizontal directions relative to the vertical axes of the individual arms, and the individual joints between the arm 3 and the arm 4, between the arm 4 and the arm 5, and between the arm 5 and the arm 6 are rotatable in the vertical axis directions of the individual arms, the direct-access endoscope attached to the front end of the arm 7 is movable in all directions, that is, three-dimensionally. The movement, that is, changes in angle between the individual arms, is sensed and recorded by the individual angle sensors provided in the individual joints. The record is inputted to a computer.

This example uses 5 movable arms, and 5 joints and angle sensors. It is preferable to use 5 or more arms and 5 each or more joints and angel sensors in order to achieve free movement of the direct-access endoscope in all directions. However, the relative positions between a joint rotatable in the horizontal direction relative to the vertical axis of an arm and a joint rotatable in the vertical axis direction of an arm are not specifically limited.

A measurement method using the inventive measuring device will now be described. Viewing from the ocular 13a of the direct-access endoscope, position of the front end 13b of the direct-access endoscope is aligned with an object to be measured, for example, one end of a tumor. Individual angles between the fixed arm 2 and the arm 3, the arm 3 and the arm 4, the arm 4 and the arm 5, the arm 5 and the arm 6, and the arm 6 and the arm 7 are sensed by the angel sensors, and inputted to the computer to record a three-dimensional position (three-dimensional coordinates) of the front end 13b of the direct-access endoscope. A projection can be attached to the tip of the direct-access endoscope, which is brought in contact with the object to be measured, to achieve simple and precision measurement.

Then, the front end 13b of the direct-access endoscope is moved to be aligned with the other end of the tumor. As the front end 13b moves, the individual arms move and angles between the individual arms are changed. The angles after movement are sensed by the angle sensors and inputted to the computer to record a three-dimensional position (three-dimensional coordinates) of the front end 13b of the direct-access endoscope after the movement. The lengths of the individual arms and the length of the direct-access endoscope are previously measured and inputted to the computer. The three-dimensional position (three-dimensional coordinates) of the front end of the direct-access endoscope can be determined from the previously-stored values and the angles between the individual arms. Then, an actual length from one end to the other of the tumor is calculated, using a conventional method known in the art, from the two three-dimensional positions (three-dimensional coordinates) of the front end 13b of the direct-access endoscope before and after the movement.

This method can measure not only the size of the tumor but also its area and distance from a specific portion. The endoscope used in the present invention is of a direct-access type which uses a lens to observe an object from outside of the body, but a fiber scope used for observation of digestive canals is not suitable for the purpose.

With the position measuring device for an endoscope according to the present invention, for example, a tumor in the human body can be observed for its shape and measured for its size, position, area and the like, and the results are obtained as numerical values.

## Claims

1. A measuring device, comprising:
(a) a plurality of arms (3 to 7) successively connected to a fixed arm (2) so that a front end of the connected plurality of arms (3 to 7) is movable three-dimensionally;
(b) angle sensors (8 to 12) provided in individual arm joints; and
(c) a computer to which the length of the individual arms (3 to 7) previously measured and the angles sensed by the angle sensors (8 to 12) are inputted;
characterized in that
(1) a direct-access endoscope (13) is connected to the front end of the connected arms (3 to 7);
(2) the computer comprises means for storing the length of the direct-access endoscope (13) previously measured; and
(3) the computer further comprises means for numerically determining the three-dimensional positions of the front end (13b) of the endoscope (13) from the lengths of the individual arms (3 to 7), the angles between the individual arms (3 to 7) and the length of the direct-access endoscope (13) and thereby to determine the numerical size and/or position and/or area and/or distance from a specific portion of an object observed by the endoscope (13).

2. The measuring device of claim 1, characterized in that said plurality of movable arms (3 to 7) comprises at least five movable arms successively connected to one another.

3. A measuring method, comprising:
(a) measuring the length of a plurality of arms (3 to 7) successively connected to a fixed arm (2) so that a front end of the connected plurality of arms (3 to 7) is movable three-dimensionally;
(b) measuring the angles between the individual arms by angle sensors (8 to 12) attached to individual joints; and
(c) inputting to a computer the length of the individual arms (3 to 7) previously measured and the angles sensed by the angle sensors (8 to 12);
characterized by
(1) measuring the length of a direct-access endoscope (13) connected to the front end of the arms (3 to 7);
(2) inputting the length of the direct-access endoscope (13) previously measured to the computer;
(3) numerically determining the three-dimensional positions of the front end (13b) of the endoscope (13) and/or the numerical size and/or position and/or area and/or distance by the computer from a specific portion of an object observed by the endoscope (13) by:
(i) aligning the front end (13b) of the endoscope (13) with one portion of the object to be measured and calculating the numerical three-dimensional position of that one portion;
(ii) aligning the front end (13b) of the endoscope (13) with another portion of the object to be measured or a specific portion and calculating the numerical three-dimensional position of that portion;
(iii)possibly repeating the steps (i) and (ii) for measuring the three-dimensional position of further portions of the object to be measured and/or the three-dimensional position of a specific portion; and
(iv) calculating the numerical size and/or area and/or position of said object and/or the numerical distance of said object from said specific portion.

## Patentansprüche

1. Meßeinrichtung, umfassend:
(a) eine Mehrzahl von Armen (3 bis 7), die hintereinander mit einem ortsfesten Arm (2) so verbunden sind, daß ein vorderes Ende der verbundenen Mehrzahl von Armen (3 bis 7) dreidimensional bewegbar ist;
(b) Winkelsensoren (8 bis 12), die in einzelnen Armgelenken vorgesehen sind; und
(c) einen Computer, in welchen die vorher gemessene Länge der einzelnen Arme (3 bis 7) und die durch die Winkelsensoren (8 bis 12) abgefühlten Winkel eingegeben werden;
dadurch **gekennzeichnet**, daß
(1) ein Direktzugangs- bzw. -einsichtsendoskop (13) mit dem vorderen Ende der verbundenen Arme (3 bis 7) verbunden ist;
(2) der Computer ein Mittel zum Speichern der vorher gemessenen Länge des Direktzugangs- bzw. -einsichtsendoskops (13) umfaßt; und
(3) der Computer weiter ein Mittel zum numerischen Bestimmen der dreidimensionalen Positionen des vorderen Endes (13b) des Endoskops (13) aus den Längen der einzelnen Arme (3 bis 7), den Winkeln zwischen den einzelnen Armen (3 bis 7) und der Länge des Direktzugangs- bzw.-einsichtsendoskops (13) und dadurch zum Bestimmen der numerischen Größe und/oder Position und/oder Fläche und/oder Entfernung von einem speziellen Teil bzw. Bereich, eines mittels des Endoskops (13) beobachteten Objekts umfaßt.

2. Meßeinrichtung nach Anspruch 1, dadurch **gekennzeichnet**, daß die Mehrzahl der bewegbaren Arme (3 bis 7) wenigstens fünf hintereinander miteinander verbundene bewegbare Arme umfaßt.

3. Meßverfahren, umfassend:
(a) Messen der Länge einer Mehrzahl von Armen (3 bis 7), die hintereinander mit einem ortsfesten Arm (2) so verbunden sind, daß ein vorderes Ende der verbundenen Mehrzahl von Armen (3 bis 7) dreidimensional bewegbar ist;
(b) Messen der Winkel zwischen den einzelnen Armen durch Winkelsensoren (8 bis 12), die an einzelnen Gelenken angebracht sind; und
(c) Eingeben der vorher gemessenen Länge der einzelnen Arme (3 bis 7) und der durch die Winkelsensoren (8 bis 12) abgefühlten Winkel in einen Computer;
**gekennzeichnet** durch
(1) Messen der Länge eines Direktzugangs- bzw. -einsichtsendoskops (13), das mit dem vorderen Ende der Arme (3 bis 7) verbunden ist;
(2) Eingeben der vorher gemessenen Länge des Direktzugangs- bzw. -einsichtsendoskops (13) in den Computer;
(3) numerisches Bestimmen der dreidimensionalen Positionen des vorderen Endes (13b) des Endoskops (13) und/oder der numerischen Größe und/oder Position und/oder Fläche und/oder Entfernung von einem speziellen Teil bzw. Bereich, eines Objekts, das mittels des Endoskops (13) beobachtet wird, mittels des Computers, durch:
(i) Ausfluchten des vorderen Endes (13b) des Endoskops (13) mit einem Teil bzw. Bereich des zu messenden Objekts und Berechnen der numerischen dreidimensionalen Position jenes einen Teils bzw. Bereichs;
(ii) Ausfluchten des vorderen Endes (13b) des Endoskops (13) mit einem anderen Teil bzw. Bereich des zu messenden Objekts oder einem speziellen Teil bzw. Bereich und Berechnen der numerischen dreidimensionalen Position jenes Teils bzw. Bereichs;
(iii) möglicherweise Wiederholen der Schritte (i) und (ii) zum Messen der dreidimensionalen Position von weiteren Teilen bzw. Bereichen des zu messenden Objekts und/oder der dreidimensionalen Position eines speziellen Teils bzw. Bereichs; und
(iv) Berechnen der numerischen Größe und/oder Fläche und/oder Position des Objekts und/oder der numerischen Entfernung des Objekts von dem speziellen Teil bzw. Bereich.

## Revendications

1. Appareil de mesure, comprenant :
(a) plusieurs bras (3 à 7) raccordés successivement à un bras fixe (2) afin qu'une extrémité avant des bras raccordés (3 à 7) soit mobile tridimensionnellement,
(b) des capteurs angulaires (8 à 12) placés aux articulations des bras individuels, et
(c) un ordinateur auquel sont transmis les longueurs des bras individuels (3 à 7) mesurées au préalable et les angles détectés par les capteurs angulaires (8 à 12),
caractérisé en ce que
(1) un endoscope (13) à accès direct est raccordé à l'extrémité avant des bras raccordés (3 à 7),
(2) l'ordinateur comporte un dispositif destiné à mémoriser la longueur de l'endoscope à accès direct (13) qui a été mesurée au préalable, et
(3) l'ordinateur comporte en outre un dispositif destiné à déterminer numériquement les positions tridimensionnelles de l'extrémité avant (13b) de l'endoscope (13) d'après les longueurs des bras individuels (3 à 7), les angles des bras individuels (3 à 7) et la longueur de l'endoscope à accès direct (13), et à déterminer la dimension et/ou position et/ou surface et/ou distance numérique à une partie spécifique d'un objet observé avec l'endoscope (13).

2. Appareil de mesure selon la revendication 1, caractérisé en ce que les bras mobiles (3 à 7) comporte au moins cinq bras mobiles raccordés successivement les uns aux autres.

3. Procédé de mesure, comprenant :
(a) la mesure des longueurs de plusieurs bras (3 à 7) raccordés successivement à un bras fixe (2) de manière qu'une extrémité avant des bras raccordés (3 à 7) soit mobile tridimensionnellement,
(b) la mesure des angles formés entre les bras individuels par des capteurs angulaires (8 à 12) fixés aux articulations individuelles, et
(c) la transmission à un ordinateur des longueurs des bras individuels (3 à 7) mesurées au préalable et des angles détectés par les capteurs angulaires (8 à 12),
caractérisé par
(1) la mesure de la longueur d'un endoscope à accès direct (13) raccordé à l'extrémité avant des bras (3 à 7),
(2) la transmission de la longueur de l'endoscope a accès direct (13) mesurée au préalable à l'ordinateur, et
(3) la détermination numérique de la position tridimensionnelle de l'extrémité avant (13b) de l'endoscope (13) et/ou de la dimension et/ou position et/ou surface et/ou distance numérique par l'ordinateur par rapport à une partie spécifique d'un objet observé avec l'endoscope (13), par :
(i) alignement de l'extrémité avant (13b) de l'endoscope (13) sur une partie de l'objet à mesurer et calcul de la position tridimensionnelle numérique de cette partie,
(ii) alignement de l'extrémité avant (13b) de l'endoscope (13) sur une autre partie de l'objet à mesurer ou une partie spécifique et calcul de la position tridimensionnelle numérique de cette partie,
(iii) répétition éventuelle des étapes (i) et (ii) pour la mesure de la position tridimensionnelle d'autres parties de l'objet à mesurer et/ou de la position tridimensionnelle d'une partie spécifique, et
(iv) calcul de la dimension et/ou surface et/ou position numérique de l'objet et/ou de la distance numérique de l'objet à la partie spécifique.
